# FASCICULE DE BREVET EUROPEEN

(11) **EP 0 949 229 B1**
(45) Date de publication et mention de la délivrance du brevet: **29.10.2003**
(21) Numéro de dépôt: 99400739.1
(22) Date de dépôt: 25.03.1999
(51) Int. Cl.: C07C 5/27

(54) **Procédé d'isomérisation des composés aromatiques à huit atomes de carbone utilisant un catalyseur contenant une zéolithe de type structural EUO**
Verfahren zur Isomerisierung von aromatischen Verbindungen mit acht Kohlenstoffatomen mit Verwendung eines Zeolith mit EUO-Struktur als Katalysator
Process for the isomerisation of aromatic compounds with eight carbon atoms using a catalyst containing a zeolite with an EUO type structure

(30) Priorité: 08.04.1998 FR 9804526
(43) Date de publication de la demande: 13.10.1999
(73) Titulaire: Institut Français du Pétrole, 92852 Rueil-Malmaison Cedex (FR)
(72) Inventeur: Alario, Fabio, 92200 Neuilly sur Seine (FR); Joly, Jean-Francois, 69006 Lyon (FR); Magne-Drisch, Julia, 38200 Vilette de Vienne (FR); Merlen, Elisabeth, 92500 Rueil-Malmaison (FR); Benazzi, Eric, 78400 Chatou (FR); Lacombe, Sylvie, 92500 Rueil Malmaison (FR)

(56) Documents cités:
- EP-A- 0 051 318
- WO-A-96/16004

## Description

La présente invention concerne le domaine des procédés d'isomérisation des composés aromatiques à huit atomes de carbone. Plus particulièrement, l'invention concerne un procédé d'isomérisation des composés aromatiques à huit atomes de carbone dans lequel le catalyseur utilisé contient une zéolithe de type strutural EUO et un élément du groupe VIII de la classification périodique des éléments (Handbook of Chemistry and Physics, 45th edition, 1964-1965).

Différentes zéolithes correspondant au type strutural EUO ont été décrites dans l'art antérieur, on peut citer par exemple la zéolithe EU-1 décrite dans le brevet EP-42226, la zéolithe TPZ-3 décrite dans le brevet EP-51318 et la zéolithe ZSM-50 décrite dans le brevet US 4 640 829.

La présente invention concerne plus spécifiquement un procédé d'isomérisation des composés aromatiques à 8 atomes de carbone dans lequel on utilise un catalyseur contenant au moins une zéolithe dé type structural EUO et au moins un métal ou un composé d'un métal du groupe VIII et dans lequel on introduit dans la zone de réaction, avec la charge contenant le ou les composés à isomériser, une phase recyclée constituée d'au moins un composé ayant un point d'ébullition d'environ 80°C à environ 135°C autre que les composés aromatiques à 8 atomes de carbone et l'hydrogène nécessaire à la réaction. Le métal du groupe VIII est habituellement choisi parmi les métaux nobles ou les composés de métaux nobles de ce groupe VIII et en particulier on utilisera du platine ou du palladium ou un composé d'au moins l'un de ces métaux et de préférence du platine ou un composé de platine. Ledit composé ayant un point d'ébullition compris entre 80 et 135°C est choisi dans le groupe constitué par le benzène, le toluène, les paraffines à 8 atomes de carbone par molécule et les naphtènes à 8 atomes de carbone par molécule.

Par rapport à l'art antérieur, le procédé selon la présente invention présente de nombreux avantages parmi lesquels on peut citer : une diminution des pertes en composés aromatiques à huit atomes de carbone par réactions secondaires parasites de dismutation, de transalkylation, d'hydrogénation et de craquage.

Selon les procédés connus d'isomérisation des composés aromatiques à huit atomes de carbone, une charge généralement pauvre en paraxylène par rapport à l'équilibre thermodynamique du mélange (c'est-à-dire dont la teneur en paraxylène est nettement inférieure à celle du mélange à l'équilibre thermodynamique à la température considérée, ce mélange comprenant au moins un composé choisi dans le groupe formé par le métaxylène, l'orthoxylène, le paraxylène et l'éthylbenzène) et généralement riche en éthylbenzène par rapport à ce même mélange à l'équilibre thermodynamique est introduite dans un réacteur contenant au moins un catalyseur, dans des conditions de température et de pression adéquates pour obtenir une composition, à la sortie dudit réacteur, en composés aromatiques à huit atomes de carbone la plus proche possible de la composition dudit mélange à l'équilibre thermodynamique à la température du réacteur.

De ce mélange, on sépare ensuite le paraxylène et éventuellement l'orthoxylène, qui sont les isomères recherchés car ils présentent un intérêt important notamment pour l'industrie des fibres synthétiques. Le métaxylène, l'éthylbenzène peuvent alors être recyclés vers l'entrée du réacteur d'isomérisation de façon à accroître la production en paraxylène et en orthoxylène. Lorsqu'on ne cherche pas à récupérer l'orthoxylène, celui-ci est recyclé avec le métaxylène et l'éthylbenzène.

Les réactions d'isomérisation des composés aromatiques à huit atomes de carbone par molécule posent cependant plusieurs problèmes engendrés par des réactions secondaires. Ainsi, outre la réaction principale d'isomérisation, on observe des réactions d'hydrogénation comme par exemple l'hydrogénation des composés aromatiques en naphtènes, des réactions d'ouverture des cycles naphténiques qui conduisent à la formation de paraffines ayant au plus le même nombre d'atomes de carbone par molécule que les naphtènes dont ils sont issus. On observe aussi des réactions de craquage, comme par exemple le craquage des paraffines qui conduisent à la formation de paraffines légères ayant typiquement de 3 à 5 atomes de carbone par molécule, des réactions de dismutation et de transalkylation qui conduisent à la production de benzène, de toluène, de composés aromatiques à 9 atomes de carbone par molécule (triméthylbenzènes par exemple) et de composés aromatiques plus lourds.

L'ensemble de ces réactions secondaires pénalise fortement les rendements en produits désirés.

La quantité de produits secondaires formés (naphtènes contenant typiquement de 5 à 8 atomes de carbone, paraffines contenant typiquement de 3 à 8 atomes de carbone, benzène, toluène, composés aromatiques à 9 et 10 atomes de carbone par molécule pour l'essentiel) dépend de la nature du catalyseur et des conditions opératoires du réacteur d'isomérisation (température, pressions partielles d'hydrogène et d'hydrocarbures, débit de charge).

Dans les procédés classiques d'isomérisation des composés aromatiques à huit atomes de carbone, un mélange de xylènes et d'éthylbenzène est mis en contact avec un catalyseur approprié, contenant généralement au moins un métal noble du groupe VIII et une zéolithe, afin d'amener le mélange de composés aromatiques à huit atomes de carbone à une composition la plus proche possible de la composition correspondant à l'équilibre thermodynamique à la température considérée.

Or, la demanderesse a trouvé de manière surprenante qu' un procédé d'isomérisation des composés aromatiques à 8 atomes de carbone dans lequel on utilise un catalyseur contenant au moins une zéolithe de type structural EUO et au moins un métal ou un composé d'un métal du groupe VIII et dans lequel on introduit dans la zone de réaction, avec la charge contenant le ou les composés à isomériser, une phase recyclée constituée d'au moins un composé ayant un point d'ébullition d'environ 80°C à environ 135°C autre que les composés aromatiques à 8 atomes de carbone et l'hydrogène nécessaire à la réaction présente des performances bien meilleures que les procédés de l'art antérieur.

Dans le procédé selon la présente invention, le catalyseur contient au moins une zéolithe de type structural EUO et au moins un métal ou un composé d'un métal du groupe VIII, il présente ainsi l'avantage d'être mis en oeuvre dans des gammes de températures plus basses, à des pressions partielles d'hydrogène plus faibles et à des PPH (poids de charge/poids de catalyseur/heure) plus élevées.

De façon préférée on utilise un catalyseur contenant une zéolithe de type structural EUO, par exemple la zéolithe EU-1 et le platine. Ce catalyseur contient au moins une zéolithe de type structural EUO, au moins en partie sous forme acide, cette zéolithe comprend du silicium et au moins un élément T choisi dans le groupe, formé par l'aluminium, le fer, le gallium et le bore, de préférence l'aluminium et le bore et dont le rapport Si/T atomique global est d'environ 5 à 100, de préférence d'environ 5 à 80, et de manière encore plus préférée d'environ 5 à 60. Ce catalyseur comprend aussi au moins une matrice (ou liant), au moins un métal ou un composé d'un métal du groupe VIII de la classification périodique des éléments. Ce catalyseur comprend aussi éventuellement au moins un métal ou un composé de métal choisi dans l'ensemble formé par les métaux ou composés de métaux des groupes IIIA et IVA de la classification périodique des éléments, et éventuellement du soufre ou au moins un composé de soufre. Le plus souvent le métal est choisi dans le groupe formé par l'étain et l'indium.

La matrice est généralement choisie dans le groupe formé par les argiles naturelles (par exemple le kaolin ou la bentonite), les argiles synthétiques, la magnésie, les alumines, les silices, les silice-alumines, l'oxyde de titane, l'oxyde de bore, la zircone, les phosphates d'aluminium, les phosphates de titane, les phosphates de zirconium, de préférence parmi les éléments du groupe formé par les alumines et les argiles. Cette matrice peut être un composé simple ou un mélange d'au moins 2 de ces composés.

La zéolite de type structural EUO, est au moins en partie, de préférence pratiquement totalement, sous forme acide, c'est-à-dire sous forme hydrogène (H⁻).

Cette zéolithe représente une teneur pondérale, de 1 à 90%, et de préférence de 3 à 60%, et de manière encore plus préférée de 4 à 40% par rapport au poids total du catalyseur.

Ce catalyseur contient aussi au moins une matrice, ou liant, assurant le complément à 100% dans le catalyseur.

Le procédé d'isomérisation des composés aromatiques à 8 atomes de carbone selon la présente invention est mis en oeuvre à une température d'environ 300°C à 500°C, de préférence d'environ 320°C à 450°C et d'une manière encore plus préférée d'environ 350°C à 420°C, à une pression partielle d'hydrogène d'environ 0,3 à 1,5 MPa, de préférence d'environ 0,4 à 1,2 MPa, à une pression totale d'environ 0,45 à 1,9 MPa, de préférence d'environ 0,6 à 1,5 MPa, à un PPH (poids de charge/ poids de catalyseur/ heure) d'environ 0,25 h⁻¹ à 30 h⁻¹, de préférence d'environ 1 h⁻¹ à 10 h⁻¹, et très souvent de 2 h⁻¹ à 6 h⁻¹.

L'examen de la littérature montre qu'il a été envisagé de réaliser des recyclages de certains constituants contenus dans l'effluent du réacteur d'isomérisation vers l'entrée dudit réacteur de façon à minimiser la production de produits secondaires.

Par exemple, les brevets US 3553276, US 3558173 et US 4255606 préconisent d'ajouter certains composés à la charge à traiter, afin de diminuer les pertes en produits secondaires.

Ainsi, le brevet US 3553276 décrit un dispositif dans lequel on recyclé du toluène de façon telle que sa concentration soit maintenue au double de la concentration que l'on obtiendrait sans ce recyclage.

Le brevet US 3558173 décrit un recyclage des naphtènes à huit atomes de carbone produits par hydrogénation des composés aromatiques correspondants.

Dans la description du brevet US 4255606, de 1 à 10% en masse par rapport à la charge totale, d'un hydrocarbure aliphatique contenant au moins 5 atomes de carbone est introduit dans la zone de réaction avec ou sans addition de toluène. Cette addition peut être assurée par un recyclage. L'hydrocarbure introduit peut aussi être un précurseur du n-pentane.

Pour minimiser la production de produits secondaires, il a égalenent été envisagé dans la demande du brevet EP-A1-051 318 d'ajouter à la charge à isomériser une certaine quantité d'amine telle que la propylamine ou le butylamine.

Le procédé d'isomérisation selon la présente invention comprend l'introduction dans la zone de réaction, avec la charge contenant les composés à isomériser et l'hydrogène nécessaire à la reaction, d'une phase recyclée constituée d'au moins un composé de point d'ébullition d'environ 80°C à 135 °C, autre que les composés aromatiques à 8 atomes de carbone, et choisi dans le groupe formé par : les paraffines à huit atomes de carbone par molécule, le benzène, le toluène, les naphtènes à huit atomes de carbone, ledit procédé comprenant la séparation de l'effluent de la zone de réaction de manière à extraire les composés ayant de un à sept atomes de carbone par molécule et les composés aromatiques ayant au moins huit atomes de carbone.

Au moins un composé choisi dans le groupe formé par les paraffines à 8 atomes de carbone par molécule, le benzène, le toluène, les naphtènes à 8 atomes de carbone est ajouté à la charge à isomériser, sous forme de recyclage, ou sous forme d'un recyclage et de composé frais en quantités telles que le pourcentage pondéral en composé ajouté par rapport à la charge totale qui entre dans le réacteur est habituellement :
- le pourcentage en masse de paraffines à huit atomes de carboné dans le cas éventuel où on en ajoute à la charge est d'environ 0,1 à 10%, de préférence d'environ 0,2 à 2%,
- le pourcentage en masse de naphtènes à huit atomes de carbone dans le cas éventuel où on en ajoute à la charge est d'environ 0,5 à 15%, et de préférence d'environ 2 à 10%,
- le pourcentage en masse de toluène dans le cas éventuel où on en ajoute à la charge est d'environ 0,01 à 5, de préférence d'environ 0,01 à 3%,
- le pourcentage en masse de benzène dans le cas éventuel où on en ajoute à la charge est d'environ 0,01 à 5%, de préférence d'environ 0,01 à 1%.

Dans le cas où plusieurs composés sont ajoutés; le pourcentage en masse de composés totaux ajoutés représente environ 0,01 à 20% et souvent environ 2 à 15 % par rapport à la charge totale qui entre dans le réacteur. Dans une forme préférée de réalisation de l'invention l'ajout comprendra au moins un naphtène.

Selon une variante préférée de réalisation de l'invention on introduit dans la zone de réaction au moins deux composés différents ayant chacun un point d'ébullition d'environ 80°C à 135 °C. Plus particulièrement, on introduit au moins un naphtène et au moins une paraffine à 8 atomes de carbone. Dans une autre variante, on introduit sans les séparer tous les composés contenus dans cette fraction liquide ayant des points d'ébullition d'environ 80°C à 135 °C. Ces composés peuvent provenir du recyclage d'une fraction liquide sortant du réacteur.

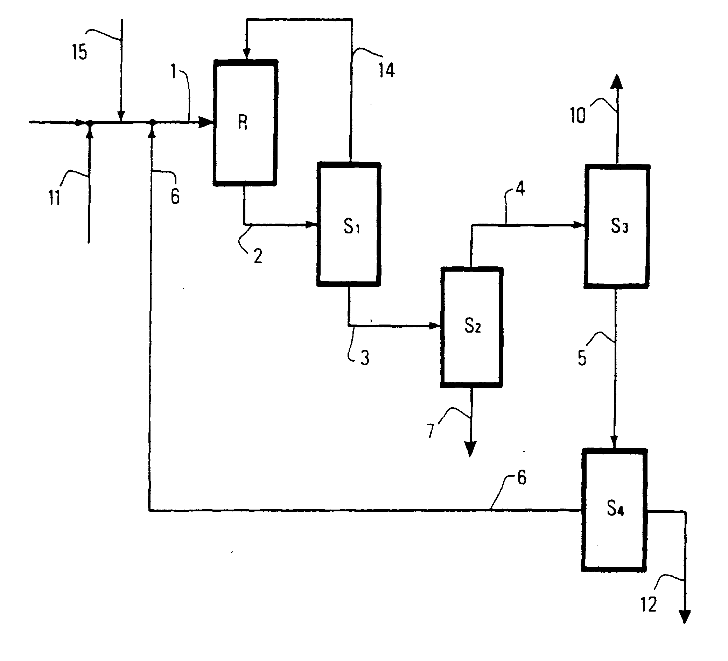

La figure 1 présente une réalisation simple du procédé selon l'invention.

Selon cette figure, la charge contenant le mélange des composés aromatiques à 8 atomes de carbone par molécule à isomériser est introduite dans le réacteur R par la ligne 1. Avant d'être injectée dans le réacteur d'isomérisation R, cette charge à isomériser est enrichie en au moins un composé choisi dans le groupe formé par les paraffines à huit atomes de carbone, le benzène, le toluène et les naphtènes à huit atomes de carbone. Ces ajouts sont effectués par un recyclage assuré par la ligne 6 et par des ajouts de composés frais par la ligne 11. Un appoint d'hydrogène sensiblement pur est assuré par la ligne 15.

Après réaction, l'effluent est envoyé dans une zone de séparation S₁ par la ligne 2. Dans S₁, on isole l'hydrogène contenu dans l'effluent et on le recycle vers l'entrée du réacteur R par la ligne 14, le reste de l'effluent est envoyé dans une zone de séparation S₂ par une ligne 3. Dans cette zone de séparation S₂, on sépare les produits de la réaction en deux fractions. Une fraction légère qui contient les paraffines, les naphtènes ainsi que les composés aromatiques les plus légers dont le benzène et le toluène est envoyée par la ligne 4 dans une zone de séparation S₃. L'autre fraction plus lourde comprenant les composés aromatiques contenant au moins huit atomes de carbone est évacuée du dispositif par la ligne 7. De cette fraction, après des étapes successives de séparation, les produits désirés, et en particulier le paraxylène, seront extraits.

Dans une zone de séparation S₃, on sépare les hydrocarbures contenant de un à sept atomes de carbone -qui sont évacués par la ligne 10- de la phase contenant les paraffines à huit atomes de carbone, les naphtènes à huit atomes de carbone, le benzène et le toluène. Ce mélange est envoyé par la ligne 5 dans une zone de séparation S₄. Dans le séparateur S₄, on choisit les quantités en paraffines à huit atomes de carbone, en benzène, en toluène et en naphtènes à huit atomes de carbone que l'on veut recycler, le mélange à recycler est ensuite envoyé par la ligne 6 en amont du réacteur où il enrichit la charge à isomériser. Une ligne 12 est prévue pour évacuer la partie du mélange comprenant des paraffines à huit atomes de carbone, des naphtènes à huit atomes de carbone, du benzène et du toluène que l'on ne désire pas recycler.

Les exemples suivants illustrent l'invention sans en limiter la portée.

Dans les exemples, les notations suivantes sont employées : "Paraffines C1-C4" pour les paraffines contenant de 1 à 4 atomes de carbone, "Paraffines C8" pour les paraffines contenant 8 atomes de carbone. "Naphtènes C6", "Naphtènes C7" et "Naphtènes C8", respectivement pour les naphtènes contenant 6, 7 et 8 atomes de carbone. "Aromatiques C9+" pour les composés aromatiques contenant au moins 9 atomes de carbone.

### Exemple 1 (conforme à l'invention)

Le catalyseur utilisé dans cet exemple est préparé selon la procédure suivante.

La matière première utilisée est une zéolithe de type structural EUO, la zéolithe EU-1, brute de synthèse, comprenant le structurant organique, du silicium et de l'aluminium, possédant un rapport atomique Si/AI global égal à 13,6 , une teneur pondérale en sodium par rapport au poids en zéolithe EU-1 sèche d'environ 1,5%, correspondant à un rapport atomique Na/Al de 0,6.

Cette zéolithe EU-1 subit tout d'abord une calcination dite sèche à 550°C sous flux d'air durant 6 heures. Puis le solide obtenu est soumis à trois échanges ioniques dans une solution de NH₄NO₃ 10N, à environ 100°C pendant 4 heures pour chaque échange.

A l'issue de ces traitements, la zéolithe EU-1 sous forme NH₄ a un rapport Si/Al atomique global égal à 18,3 , une teneur pondérale en sodium par rapport au poids de zéolithe EU-1 sèche de 50 ppm.

La zéolithe EU-1 est ensuite mise en forme par extrusion avec un gel d'alumine de manière à obtenir, après séchage et calcination sous air sec, le support S1 constitué d'extrudés de 1,4 mm de diamètre, qui contient en poids 10 % de zéolithe EU-1 sous forme H et 90 % d'alumine.

Le support S1 ainsi obtenu est soumis à un échange anionique avec de l'acide hexachloroplatinique en présence d'un agent compétiteur (acide chlorhydrique), de manière à introduire 0,3% poids de platine par rapport au catalyseur. Le solide humide est ensuite séché à 120°C pendant 12 heures et calciné sous un débit d'air sec à la température de 500°C pendant une heure.

Le catalyseur C1 ainsi obtenu contient en poids 10,0% de zéolithe EU-1 sous forme H, 89,7% d'alumine et 0,29% de platine.

On utilise ce catalyseur dans les conditions opératoires suivantes : une pression totale de 0,9 MPa, une température de 380 °C et un PPH de 3 h.

L'effluent liquide sortant du réacteur est distillé de façon à récupérer la fraction dont l'intervalle de distillation est de 80°C à 135°C. Cette fraction est alors ajoutée à la charge à isomériser. Le mélange ainsi obtenu est utilisé comme charge pour le réacteur.

Les compositions de la charge et des produits obtenus sont donnés dans le tableau 1 ci-dessous :

**Tableau 1**

| Composés | Charge (% masse) | Produits (% masse) |
|---|---|---|
| Paraffines C1-C4 | 0 | 0,53 |
| i-Pentane | 0 | 0,05 |
| n-Pentane | 0 | 0,12 |
| Benzène | 0,04 | 0,11 |
| Toluène | 0,53 | 0,65 |
| o-Xylène | 21,85 | 19,04 |
| m-Xylène | 51,63 | 40,81 |
| p-Xylène | 2,63 | 17,41 |
| Ethylbenzène | 14,22 | 11,45 |
| Naphtènes C6 | 0,14 | 0,12 |
| Naphtènes C7 | 0 | 0,17 |
| Paraffines C8 | 0,23 | 0,36 |
| Naphtènes C8 | 8,56 | 8,99 |
| Aromatiques C9+ | 0,17 | 0,18 |

### Exemple 2 (non conforme à l'invention)

La zéolithe de départ est une mordénite de forme sodique, de rapport Si/AI égal à 5,2 et de volume de maille élémentaire de 2,794 nm³. La zéolithe est soumise à trois échanges ioniques dans une solution de NH₄NO₃ 10N à environ 100°C pendant 4 heures. Le solide ainsi obtenu contient 25 ppm de sodium.

Cette zéolithe de forme hydrogène est ensuite mise en forme par extrusion avec un gel d'alumine de manière à obtenir, après séchage et calcination sous air sec, un catalyseur qui contient 15 % en masse de zéolithe mordénite forme hydrogène et 85 % en masse d'alumine.

Ce catalyseur est soumis à un échange anionique avec de l'acide hexachloroplatinique en présence d'un agent compétiteur (acide chlorhydrique), de manière à déposer 0,3 % en masse de platine par rapport au catalyseur. Le solide humide est ensuite séché à 120°C pendant 12 heures et calciné sous un débit d'air sec à la température de 500°C pendant 1 heure. Le catalyseur ainsi obtenu contient 15,0 % en masse de mordénite forme hydrogène, 84.7 % en masse d'alumine et 0,29 % en masse de platine.

Ce catalyseur est utilisé dans les mêmes conditions opératoires que celles de l'exemple 1.

De façon à pouvoir comparer les peformances des catalyseurs à base de zéolithe EU-1 et de mordénite on utlise dans l'exemple 2 la même charge que celle du tableau 1 de l'exemple 1.

Les compositions de la charge et des produits obtenus sont donnés dans le tableau 2 ci-dessous

**Tableau 2**

| Composés | Charge (% masse) | Produits (% masse) |
|---|---|---|
| Paraffines C1-C4 | 0 | 0,263 |
| i-Pentane | 0 | 0 |
| n-Pentane | 0 | 0,078 |
| Benzène | 0,04 | 0,294 |
| Toluène | 0,53 | 1,075 |
| o-Xylène | 21,85 | 18,977 |
| m-Xylène | 51,63 | 42,142 |
| p-Xylène | 2,63 | 16,505 |
| Ethylbenzène | 14,22 | 10,405 |
| Naphtènes C6 | 0,14 | 0,1 18 |
| Naphtènes C7 | 0 | 0,233 |
| Paraffines C8 | 0,23 | 0,414 |
| Naphtènes C8 | 8,56 | 8,2 |
| Aromatiques C9+ | 0,17 | 1,3 |

Les résultats du tableau 2 ci-dessus montrent très clairement l'intérêt qu'il y a à utiliser le catalyseur à base de zéolithe de type structural EUO.

La teneur en paraxylène dans l'effluent est supérieure, et est de 17,41% en masse lorsqu'on utilise le procédé en présence d'un catalyseur contenant une zéolithe de type structural EUO selon l'invention au lieu de 16,50% en masse lorsqu'on utilise le catalyseur à base de mordénite.

Le rendement en composés aromatiques à huit atomes de carbone + naphtènes à huit atomes de carbone obtenu en présence du catalyseur contenant une zéolithe de type structural EUO est égal à 98,8% à comparer à 97,31% obtenu sur catalyseur à base de mordénite. Le procédé selon la présente invention en présence d'un catalyseur à base de zéolithe de type structural EUO conduit à un gain d'activité et de sélectivité par rapport au système de référence à base de mordénite.

### Exemple 3 (conforme à l'invention)

On utilise le même catalyseur que celui de l'exemple 1.

On utilise le catalyseur dans les conditions opératoires suivantes : une pression totale de 9 bar absolus, une température de 385°C et une PPH de 3 h .

La charge à convertir est un mélange de composés aromatiques à 8 atomes de carbone et de naphtènes à 8 atomes de carbone.
La composition en masse de la charge et des produits obtenus est donnée dans le tableau 3.

**Tableau 3**

| Composés | Charge (% masse) | Produits (% masse) |
|---|---|---|
| Paraffines C1-C4 | 0 | 0,51 |
| i-Pentane | 0 | 0,14 |
| n-Pentane | 0 | 0,06 |
| Benzène | 0 | 0,07 |
| Toluène | 0 | 0,38 |
| o-Xylène | 23,85 | 19,41 |
| m-Xylène | 53,38 | 42,22 |
| p-Xylène | 1,49 | 18,5 |
| Ethylbenzène | 13,26 | 9,47 |
| Naphtènes C6 | 0 | 0,04 |
| Naphtènes C7 | 0 | 0,11 |
| Paraffines C8 | 0 | 0,21 |
| Naphtènes C8 | 8,02 | 8,52 |
| Aromatiques C9+ | 0 | 0,37 |

### Exemple 4 (non conforme à l'invention)

On utilise le même catalyseur que celui de l'exemple 2.

Ce catalyseur est utilisé dans les mêmes conditions opératoires que celles de l'exemple 3.

De façon à pouvoir comparer les performances des catalyseurs à base de zéolithe EU-1 et de mordénite on utlise dans l'exemple 4 la même charge que celle du tableau 3 de l'exemple 3.

Les compositions de la charge et des produits obtenus sont donnés dans le tableau 4 ci-dessous.

**Tableau 4**

| Composés | Charge (% masse) | Produits (% masse) |
|---|---|---|
| Paraffines C1-C4 | 0 | 0,42 |
| i-Pentane | 0 | 0,16 |
| n-Pentane | 0 | 0,05 |
| Benzène | 0 | 0,19 |
| Toluène | 0 | 0,68 |
| o-Xylène | 23,85 | 19,43 |
| m-Xylène | 53,38 | 42,38 |
| p-Xylène | 1,49 | 17,37 |
| Ethylbenzène | 13,26 | 9,54 |
| Naphtènes C6 | 0 | 0,08 |
| Naphtènes C7 | 0 | 0,16 |
| Paraffines C8 | 0 | 0,26 |
| Naphtènes C8 | 8,02 | 7,99 |
| Aromatiques C9+ | 0 | 1,29 |

Les résultats du tableau 4 ci-dessus montrent très clairement l'intérêt qu'il y a à utiliser le catalyseur à base de zéolithe de type structural EUO.

La teneur en paraxylène dans l'effluent est supérieure, et est de 18,50% en masse lorsqu'on utilise le procédé en présence d'un catalyseur contenant une zéolithe de type structural EUO selon l'invention au lieu de 17.37% en masse lorsqu'on utilise le catalyseur à base de mordénite.

Le rendement en composés aromatiques à huit atomes de carbone et en naphtènes à huit atomes de carbone obtenu en présence du catalyseur contenant une zéolithe de type structural EUO est égal à 98,1% à comparer à 96,7% obtenu sur catalyseur à base de mordénite. Le procédé selon la présente invention en présence d'un catalyseur à base de zéolithe de type structural EUO conduit à un gain d'activité et de sélectivité par rapport au système de référence à base de mordénite.

## Revendications

1. Procédé d'isomérisation des composés aromatiques à 8 atomes de carbone **caractérisé en ce que** le catalyseur utilisé contient au moins une zéolithe de type structural EUO et au moins un métal ou un composé de métal du groupe VIII et **en ce que** l'on introduit dans la zone de réaction, avec la charge contenant le ou les composé(s) à isomériser, de l'hydrogène et une phase recyclée constituée d'au moins un composé dont le point d'ébullition est compris entre 80 et 135°C autre que les composés aromatiques à 8 atomes de carbone, ledit composé étant choisi dans le groupe constitué par le benzène, le toluène, les paraffines à 8 atomes de carbone par molécule et les naphtènes à 8 atomes de carbone par molécule, ledit procédé comprenant la séparation de l'effluent de la zone de réaction de manière à extraire les composés ayant de un à sept atomes de carbone par molécule et les composés aromatiques ayant au moins huit atomes de carbone.

2. Procédé d'isomérisation des composés aromatiques à 8 atomes de carbone selon la revendication 1 **caractérisé en ce que** la zéolithe de type structural EUO contenue dans le catalyseur est choisie dans le groupe formé par les EU-1, les zéolithes TPZ-3 et les zéolithes ZSM-50.

3. Procédé d'isomérisation des composés aromatiques à 8 atomes de carbone selon la revendication 1 ou 2 **caractérisé en ce que** le catalyseur utilisé comprend au moins une matrice, au moins un élément du groupe VIII et au moins une zéolithe de type structural EUO, cette zéolithe contenant du silicium et au moins un élément T choisi dans le groupe formé par l'aluminium, le fer, le gallium et le bore, tel que le rapport Si/T atomique global est d'environ 5 à 100, ladite zéolithe étant en outre au moins en partie sous forme acide.

4. Procédé d'isomérisation des composés aromatiques à 8 atomes de carbone selon l'une des revendications 1 à 3 **caractérisé en ce que** la zéolithe de type structural EUO contenue dans la catalyseur est une zéolithe de type EU-1.

5. Procédé d'isomérisation des composés aromatiques à 8 atomes de carbone selon l'une des revendications 1 à 4 **caractérisé en ce qu'**il est mis en oeuvre à une température d'environ 300°C à 500°C, à une pression partielle d'hydrogène d'environ 0,3 MPa à 1,5 MPa, à une pression totale d'environ 0,45 à 1,9 MPa et à un PPH (poids de charge/ poids de catalyseur/ heure) d'environ 0,25 à 30 h ⁻¹.

6. Procédé d'isomérisation des composés aromatiques à 8 atomes de carbone selon l'une des revendications 1 à 5 **caractérisé en ce qu'**un mélange de composés frais de point d'ébullition compris entre 80 et 135°C est introduit dans la zone de réaction, en plus de la phase recyclée.

7. Procédé d'isomérisation des composés aromatiques à 8 atomes de carbone selon l'une des revendications 1 à 6 **caractérisé en ce que** le pourcentage en masse de paraffines à huit atomes de carbone ajoutées par rapport à la charge totale qui entre dans le réacteur est d'environ 0,1 à 10 %.

8. Procédé d'isomérisation des composés aromatiques à 8 atomes de carbone selon l'une des revendications 1 à 7 **caractérisé en ce que** le pourcentage en masse de naphtènes à huit atomes de carbone ajoutés par rapport à la charge totale qui entre dans le réacteur est d'environ 0,5 à 15 %.

9. Procédé d'isomérisation des composés aromatiques à 8 atomes de carbone selon l'une des revendications 1 à 8 **caractérisé en ce que** le pourcentage en masse de toluène ajouté par rapport à la charge totale qui entre dans le réacteur est d'environ 0,01 à 5 %.

10. Procédé d'isomérisation des composés aromatiques à 8 atomes de carbone selon l'une des revendications 1 à 9 **caractérisé en ce que** le pourcentage en masse de benzène ajouté par rapport à la charge totale qui entre dans le réacteur est d'environ 0,01 à 5 %.

## Patentansprüche

1. Verfahren zur Isomerisierung der aromatischen Verbindungen mit 8 Kohlenstoffatomen, **dadurch gekennzeichnet, dass** der verwendete Katalysator wenigstens einen Zeolith vom Strukturtyp EUO und wenigstens ein Metall oder eine Verbindung eines Metalls der Gruppe VIII enthält und dadurch , dass man in die Reaktionszone mit der Beschickung, welche die zu isomerisierende(n) Verbindung(en) enthält, Wasserstoff und eine rezyklierte Phase einführt, die aus wenigstens einer Verbindung besteht, deren Siedepunkt zwischen 80 und 135°C liegt, anders als die aromatischen Verbindungen mit 8 Kohlenstoffatomen pro Molekül, wobei die Verbindung aus der Gruppe gewählt ist, die aus Benzol, Toluol, den Paraffinen mit 8 Kohlenstoffatomen pro Molekül und den Naphtenen mit 8 Kohlenstoffatomen pro Molekül besteht, wobei das Verfahren die Trennung des Abstroms der Reaktionszone derart umfasst, dass die Verbindungen mit sieben Kohlenstoffatomen pro Molekül und die aromatischen Verbindungen mit wenigstens acht Kohlenstoffatomen pro Molekül abgezogen werden.

2. Verfahren zur Isomerisierung der aromatischen Verbindungen mit 8 Kohlenstoffatomen nach Anspruch 1, **dadurch gekennzeichnet, dass** der in dem Katalysator enthaltene Zeolith vom Strukturtyp EUO aus der Gruppe gewählt ist, die gebildet wird durch die EU-1, die Zeolithe TPZ-3 und die Zeolithe ZSM-50.

3. Verfahren zur Isomerisierung der aromatischen Verbindungen mit 8 Kohlenstoffatomen nach Anspruch 1 oder 2, **dadurch gekennzeichnet, dass** der verwendete Katalysator wenigstens eine Matrix, wenigstens ein Element der Gruppe VIII und wenigstens einen Zeolith vom Strukturtyp EUO umfasst, wobei dieser Zeolith Silizium und wenigstens ein Element T enthält, das aus der Gruppe gewählt ist, die gebildet wird durch Aluminium, Eisen, Gallium und Bor, derart, dass das Si/T-Atomgesamtverhältnis etwa 5 bis 100 ist, wobei der Zeolith außerdem wenigstens teilweise in Säureform vorliegt.

4. Verfahren zur Isomerisierung der aromatischen Verbindungen mit 8 Kohlenstoffatomen nach einem der Ansprüche 1 bis 3, **dadurch gekennzeichnet, dass** der in dem Katalysator enthaltene Zeolith vom Strukturtyp EUO ein Zeolith vom EU-1-Typ ist.

5. Verfahren zur Isomerisierung der aromatischen Verbindungen mit 8 Kohlenstoffatomen nach einem der Ansprüche 1 bis 4, **dadurch gekennzeichnet, dass** es bei einer Temperatur von etwa 300°C bis 500°C, bei einem Wasserstoffpartialdruck von etwa 0,3 MPa bis 1,5 MPa, bei einem Gesamtdruck von etwa 0,45 bis 1,9 MPa und einer PPH (Beschickungsgewicht/ Katalysatorgewicht/Stunde) von etwa 0,25 bis 30 h⁻¹ durchgeführt wird.

6. Verfahren zur Isomerisierung der aromatischen Verbindungen mit 8 Kohlenstoffatomen nach einem der Ansprüche 1 bis 5, **dadurch gekennzeichnet, dass** ein Gemisch frischer Verbindungen mit einem Siedepunkt zwischen 80 und 135°C in die Reaktionszone zusätzlich zu der rezyklierten Phase eingeführt wird.

7. Verfahren zur Isomerisierung der aromatischen Verbindungen mit 8 Kohlenstoffatomen nach einem der Ansprüche 1 bis 6, **dadurch gekennzeichnet, dass** der Masseprozentanteil zugegebener Paraffine mit 8 Kohlenstoffatomen pro Molekül im Verhältnis zur Gesamtbeschickung, die in den Reaktor eintritt, etwa 0,1 bis 10% ist.

8. Verfahren zur Isomerisierung der aromatischen Verbindungen mit 8 Kohlenstoffatomen nach einem der Ansprüche 1 bis 7, **dadurch gekennzeichnet, dass** der Masseprozentanteil zugegebener Naphtene mit 8 Kohlenstoffatomen pro Molekül im Verhältnis zur Gesamtbeschickung, die in den Reaktor eintritt, etwa 0,5 bis 15% ist.

9. Verfahren zur Isomerisierung der aromatischen Verbindungen mit 8 Kohlenstoffatomen nach einem der Ansprüche 1 bis 8, **dadurch gekennzeichnet, dass** der Masseprozentanteil zugegebenen Toluols im Verhältnis zur Gesamtbeschickung, die in den Reaktor eintritt, etwa 0,01 bis 5% ist.

10. Verfahren zur Isomerisierung der aromatischen Verbindungen mit 8 Kohlenstoffatomen nach einem der Ansprüche 1 bis 9, **dadurch gekennzeichnet, dass** der Masseprozentanteil zugegebenen Benzols im Verhältnis zur Gesamtbeschickung, die in den Reaktor eintritt, etwa 0,01 bis 5% ist.

## Claims

1. A process for isomerising aromatic compounds containing eight carbon atoms, **characterized in that** the catalyst used contains at least one zeolite with structure type EUO and at least one metal or a compound of a metal from group VIII, and **in that** hydrogen and a recycled phase constituted of at least one compound with a boiling point of 80°C to 135°C other than the aromatic compounds containing eight carbon atoms are introduced into the reaction zone with the feed containing the compound or compounds to be isomerised, said compound with a boiling point of 80°C to 135°C being selected from the group formed by paraffins containing eight carbon atoms, naphthenes containing eight carbon atoms, toluene and benzene, said process comprising the separation of the effluent of the reaction zone so as to extract coumpounds containing one to seven carbon atoms per molecule and aromatic coumpounds with at least eight carbon atoms per molecule.

2. A process for isomerising aromatic compounds containing eight carbon atoms according to claim 1, **characterized in that** the zeolite with structure EUO contained in the catalyst is selected from the group formed by EU-1 zeolites, TPZ-3 zeolites and ZSM-50 zeolites.

3. A process for isomerising aromatic compounds containing eight carbon atoms according to claim 1 or claim 2, **characterized in that** the catalyst used comprises at least one matrix, at least one group VIII element and at least one zeolite with structure type EUO, said zeolite containing silicon and at least one element T selected from the group formed by aluminium, iron, gallium and boron, such that the global Si/T atomic ratio is about 5 to 100, said zeolite also being at least partially in its acid form.

4. A process for isomerising aromatic compounds containing eight carbon atoms according to any one of claims 1 to 3, **characterized in that** the zeolite with structure type EUO contained in the catalyst is an EU-1 type zeolite.

5. A process for isomerising aromatic compounds containing eight carbon atoms according to any one of claims 1 to 4, **characterized in that** it is carried out at a temperature of about 300°C to 500°C, at a partial pressure of hydrogen of about 0.3 MPa to 0.15 MPa, at a total pressure of about 0.45 to 1.9 MPa and at an HSV (weight of feed/weight of catalyst/hour) of about 0.25 to 30 h⁻¹.

6. A process for isomerising aromatic compounds containing eight carbon atoms according to any one of claims 1 to 5, **characterized in that** a mixture of fresh coumpounds with a boiling point of 80°C to 135°C is introduced in the reaction zone, besides the recycled phase.

7. A process for isomerising aromatic compounds containing eight carbon atoms according to any one of claims 1 to 6, **characterized in that** the percentage by weight of paraffins containing eight carbon atoms added with respect to the total feed entering the reactor is about 0.1% to 10%.

8. A process for isomerising aromatic compounds containing eight carbon atoms according to any one of claims 1 to 7, **characterized in that** the percentage by weight of naphthenes containing eight carbon atoms added with respect to the total feed entering the reactor is about 0.5% to 15%.

9. A process for isomerising aromatic compounds containing eight carbon atoms according to any one of claims 1 to 8, **characterized in that** the percentage by weight of toluene added with respect to the total feed entering the reactor is about 0.01% to 5%.

10. A process for isomerising aromatic compounds containing eight carbon atoms according to any one of claims 1 to 9, **characterized in that** the percentage by weight of benzene with respect to the total feed entering the reactor is about 0.01% to 5%.
